# EUROPEAN PATENT APPLICATION

(11) **EP 4 650 776 A1**
(43) Date of publication of application: **19.11.2025**
(21) Application number: 24728004.3
(22) Date of filing: 05.01.2024
(51) Int. Cl.: G01N 33/543, G01N 33/549

(54) **LATERAL FLOW ASSAY STRIP COMPRISING POLYCRYSTALLINE STRUCTURE, AND METHOD FOR PRODUCING SAME**

(30) Priority: 09.01.2023 KR 20230002977
(71) Applicant: KOREA INSTITUTE OF MATERIALS SCIENCE, Changwon-si, Gyeongsangnam-do 51508 (KR)
(72) Inventor: JUNG, Ho-sang, Changwon-si Gyeongsangnam-do 51480 (KR); KIM, Dong-ho, Changwon-si Gyeongsangnam-do 51382 (KR); PARK, Sung-gyu, Changwon-si Gyeongsangnam-do 51531 (KR); LEE, Min-young, Seoul 06376 (KR)
(74) Representative: Kador & Partner Part mbB
(86) International application number: PCT/KR2024/000310
(87) International publication number: WO 2024/151021

(57) **Abstract**

The present disclosure provides a lateral flow assay strip having a polycrystalline structure and a preparation method thereof. More specifically, the present disclosure provides a lateral flow assay strip using a technique of growing a plasmonic polycrystalline structure and having a polycrystalline structure to allow on-stie diagnosis with high sensitivity and simultaneously detect a plurality of targets and an effective preparation method thereof.

## Description

### BACKGROUND

### 1. Technical Field

The present disclosure relates to a lateral flow assay strip having a polycrystalline structure and a preparation method thereof. More specifically, the present disclosure relates to a lateral flow assay strip using a technique of growing a plasmonic polycrystalline structure and having a polycrystalline structure to allow on-stie diagnosis with high sensitivity and simultaneously detect a plurality of targets and an effective preparation method thereof.

### 2. Description of Related Art

A diagnosis method and a diagnosis instrument, where a small amount of materials, such as nucleic acid or protein, included in biological specimen, such as blood, urine, saliva, or a tear drop, is measured qualitatively or quantitatively, have been developed continuously and rapidly. Since the first introduction of radioactive immunoassay (RIA) using a radioisotope in 1950's, an enzyme immunoassay (enzyme-linked immunosorbent assay, ELISA) has been developed and improved in 70's and 80's.

A recently developed, representative method of detection of a protein or a nucleic acid is lateral flow assay (LFA), which is based on chromatography. A lateral flow assay is a diagnosis method, where an antigen-antibody reaction is generally used for intuitive determination of infection of a disease on membrane, and uses a phenomenon, where, as a specimen such as blood or urine proceeds to a porous membrane through a capillary action, a probe is fixed on a certain portion of a membrane by an antigen-antibody reaction.

A conventional test strip using lateral flow assay is consisted of a specimen pad for introducing a specimen, a conjugation pad where a biochemical reaction occurs between a specimen and a probe, a test pad where a immunity test proceeds as the sample flows by a capillary phenomenon, and an absorption pad located downstream of the test pad so that a lateral flow is maintained by absorbing an excess amount of specimen that are disposed in one direction. In addition, the test pad is provided with a detection line, where an immune reaction occurs with a target, and a control line.

Such lateral flow assay is widely used in various fields such as pregnancy diagnosis, cancer diagnosis, presence of other specific proteins or genes, or detection of microorganism.

However, there is a shortcoming in a practical on-site diagnosis (point of care, POC) that, on many occasions, it is difficult to detect at a high sensitivity when a concentration of a biological specimen is low. Specifically, the circumstance is that there are only few techniques for detection of a non-label biological specimen at a high sensitivity.

In addition, a conventional lateral flow assay strip has a shortcoming in simultaneously detecting a plurality of targets at a high sensitivity.

Furthermore, there has been a challenge in preparation of a conventional lateral flow diagnosis strip that it is not easy to form a multi-detection line, control spacing of detection lines, and form detection lines for a heterogeneous material.

As background of the present disclosure, a biosensor strip using a redox cycling, where a product produced by a catalyst label is participated, is described in Korean Patent No. 10-2014-0110795.

### SUMMARY

A purpose of the present disclosure is to provide a lateral flow assay strip capable of on-site diagnosis of a non-label specimen at a high sensitivity.

Another purpose of the present disclosure is to provide a lateral assay strip capable of simultaneously detecting various species of targets at a high sensitivity.

Another purpose of the present disclosure is to provide a preparation method of a lateral flow assay strip where it is easy to form a multi-detection line and a detection line for a heterogeneous material and control spacing of detection lines.

Purposes of the present disclosure are not limited to the purposes stated above. Other unstated purposes should be clearly understood from the detailed description.

According to one aspect, a lateral flow assay strip including a strip member having a test area including at least one detection part, where a biochemical reaction occurs between a target included in a specimen and a probe as the specimen flows by a capillary action, is provided. The detection part is consisted of a plurality of clusters of nanoparticles to include a polycrystalline structure having a plurality of grain boundaries.

According to one embodiment, the strip member may include a specimen area for introducing a specimen; the test area; and an absorption area for absorbing an excess amount of a specimen to maintain lateral flow that are placed in one direction.

According to one embodiment, the lateral flow assay strip may be for Surface Enhanced Raman Spectroscopy (SERS), fluorescence, or plasmon-enhanced fluorescence (PEF) analysis.

According to one embodiment, the nanoparticle may be consisted of at least one species of Au, Ag, and Pt.

According to one embodiment, the lateral flow assay strip may have the polycrystalline structure combined with a genetic probe.

According to one embodiment, the detection part may include at least one of the following: i) at least two vertical detection lines positioned perpendicular to a direction of a specimen flow; ii) at least two horizontal detection lines positioned parallel to a direction of a specimen flow; and iii) at least one vertical detection line positioned perpendicular to a direction of a specimen flow and at least one horizontal detection line positioned parallel to a direction of a specimen flow.

According to one embodiment, the detection part may include at least one vertical detection line positioned perpendicular to a direction of a specimen flow and at least one detection part in a form of a circle, an ellipse, or a polygonal shape positioned behind the vertical detection line with respect to the direction of a specimen flow.

According to one embodiment, a hydrophobically treated area treated with hydrophobic treatment partially around the detection part so that a specimen passed through the vertical detection line is gathered to the detection part may be provided.

According to one embodiment, the detection part may further include a detection part of a heterogeneous material.

According to one embodiment, the detection part may include a first detection part with formation of a graphene oxide layer and at least two second detection parts with formation of the polycrystalline structure, the first detection part combined with a fluorescent particle labeled genetic probe with fluorescence quenched, the second detection parts combined with a probe to combine with the fluorescent particle to recover fluorescence as the genetic probe combines with a miRNA target molecule so that a lateral flow assay strip of the present disclosure can be used as a multiplex sensor.

According to one embodiment, a probe to be attached to each second detection part may be anti-FAM, anti-TAMRA, anti-Methylene Blue, anti-Cy5, anti-Cy3, anti-Alexa Fluor 488, anti-Rhodamine Red, anti-Texas Red, or anti-fluorescein/Oregon Green.

According to one embodiment, the strip member may be consisted of at least one species of Cellulose Acetate (CA), Mixed Cellulose Ester (MCE), Nitro Cellulose (NC), CN95, and a filter membrane composed of a synthetic polymer.

According to one embodiment, the target may be selected from at least one species of cells, metabolites, proteins, nucleic acids, DNAs, RNAs, enzymes, organic molecules, viruses, extracellular vesicles, microvesicles, exosomes, and fat in urine, saliva, sweat, blood and tear drops.

According to one embodiment, the detection part may be provided with a first detection part and a second detection part orderly positioned with respect to a direction of the specimen flow, the first detection part, which first to be contact with the specimen, having a polycrystalline structure combined with a probe peptide to be decomposed by Matrix Metalloproteinase (MMP), the second detection part combined with a probe for detecting the decomposed probe peptide, so that the lateral flow assay strip of the present disclosure may be used as a MMP sensor.

According to one embodiment, the detection part may be provided with a first detection part, and a second detection part and a third detection part positioned behind the first detection part, the second detection part and the third detection part positioned parallel to each other, the first detection part having a polycrystalline structure combined with a gene reacting with an intercalation dye as a first control, the second detection part having a polycrystalline structure combined with an antibody that can combine with Me-DNA, the third detection part having a polycrystalline structure combined with the genetic probe, so that the lateral flow assay strip of the present disclosure can be used as a Me-DNA sensor.

According to one embodiment, the detection part may be provided with a first detection part, a second detection part and a third detection part horizontally positioned with respect to a direction of the specimen flow, the first detection part, the second detection part and the third detection part positioned parallel to one another, the first detection part having a polycrystalline structure combined with a gene reacting with an intercalation dye as a first control, the second detection part having a polycrystalline structure combined with an antibody that can combine with Me-DNA, the third detection part having a polycrystalline structure combined with the genetic probe, so that the lateral flow assay strip of the present disclosure can be used as a Me-DNA sensor.

According to another aspect, a preparation method of a lateral flow assay strip including A-1) preparing a strip member for preparing a strip member for lateral flow assay; A-2) attaching a tape for attaching a tape to the strip member except for an area to be patterned; A-3) forming a polycrystalline structure for forming a polycrystalline structure having a plurality of grain boundaries by soaking the strip member in a composition including a solution of a noble metal precursor and a reducing agent to form a plurality of clusters of nanoparticles on the strip member; and A-4) removing the tape for removing the tape from the strip member, is provided.

According to another aspect, a preparation method of a lateral flow assay strip including B-1) preparing a strip member for preparing a strip member for lateral flow assay; B-2) forming a polycrystalline structure for forming a polycrystalline structure having a plurality of grain boundaries by soaking the strip member in a composition including a solution of a noble metal precursor and a reducing agent to form a plurality of clusters of nanoparticles on the strip member; B-3) attaching a tape for attaching a tape to the strip member except for an area to be patterned; and B-4) removing the tape for removing the tape from the strip member, is provided.

According to another aspect, a preparation method of a lateral flow assay strip including C-1) preparing a strip member for preparing a strip member for lateral flow assay; C-2) forming a graphene oxide layer on the strip member by using a graphene oxide solution; C-3) attaching a first tape for attaching a tape to the strip member except for an area for the graphene oxide to be patterned; C-4) removing the first tape for removing the tape from the strip member; C-5) attaching a second tape for attaching a tape to the strip member except for an area for a polycrystalline structure to be patterned; C-6) forming a polycrystalline structure for forming a polycrystalline structure having a plurality of grain boundaries by soaking the strip member in a composition including a solution of a noble metal precursor and a reducing agent to form a plurality of clusters of nanoparticles on the strip member; and C-7) removing the second tape for removing the second tape from the strip member, is provided.

According to another aspect, a preparation method of a lateral flow assay strip including D-1) preparing a strip member for preparing a strip member for lateral flow assay; D-2) forming a polycrystalline structure for forming a polycrystalline structure having a plurality of grain boundaries by soaking the strip member in a composition including a solution of a noble metal precursor and a reducing agent to form a plurality of clusters of nanoparticles on the strip member; D-3) attaching a first tape for attaching a tape to the strip member except for an area for a polycrystalline structure to be patterned; D-4) removing the first tape for removing the tape from the strip member; D-5) attaching a second tape for attaching a tape to the strip member except for an area for graphene oxide to be patterned; D-6) forming a graphene oxide layer on the strip member by using a graphene oxide solution; and D-7) removing the second tape for removing the tape from the strip member, is provided.

According to one embodiment of the present disclosure, a lateral flow assay strip of the present disclosure includes a plasmonic polycrystalline structure to allow on-site diagnosis of a non-label biological specimen at a high sensitivity.

According to one embodiment of the present disclosure, the lateral flow assay strip of the present disclosure includes a plurality of detection lines including a plasmonic polycrystalline structure to allow simultaneous detection of heterogeneous targets at a high sensitivity.

According to one embodiment of the present disclosure, a polycrystalline structure is patterned by a solution process to easily form a multi-detection line and a detection line of a heterogeneous material and effectively control spacing of detection lines in a preparation method of a lateral flow assay strip of the present disclosure.

### BRIEF DESCRIPTION OF THE DRAWINGS

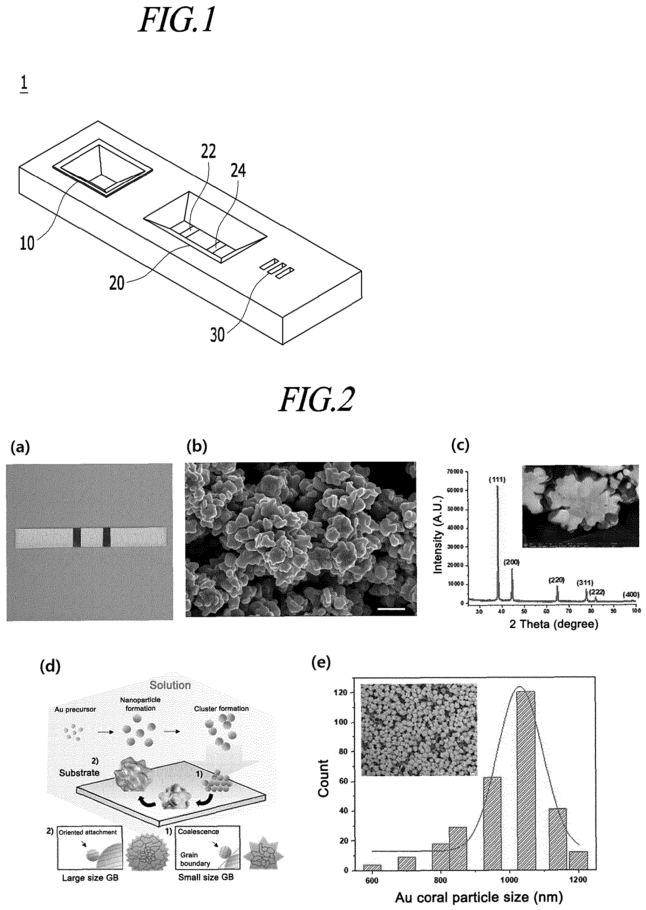

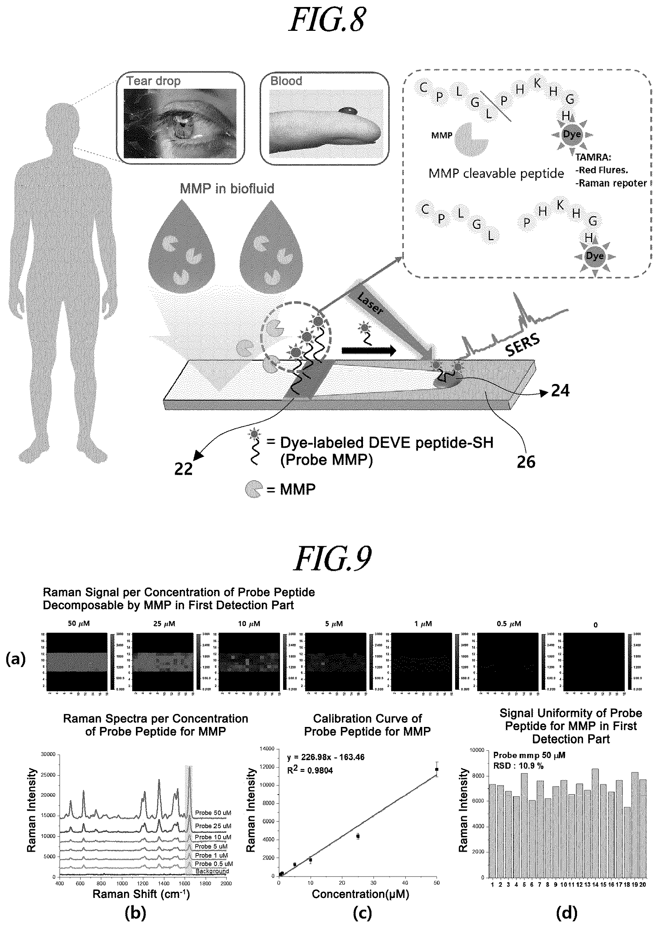

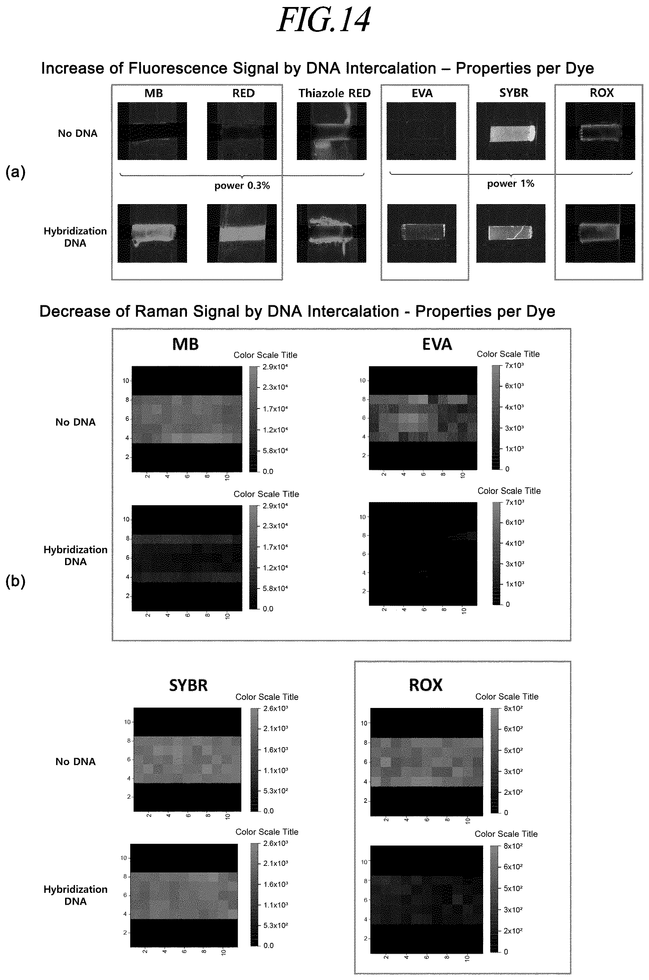

FIG. 1 schematically illustrates a lateral flow assay strip including a polycrystalline structure in a detection line according to one embodiment of the present disclosure.
(a) of FIG. 2 illustrates a detection part prepared with a strip member of cellulose acetate according to one embodiment of the present disclosure. (b) to (e) of FIG. 2 illustrate a SEM photograph of a detection part in (a) of FIG. 2 (see (b) of FIG. 2), a polycrystalline verification result (see (c) of FIG. 2), a nanoparticle formation mechanism (see (d) of FIG. 2), and a nanoparticle distribution graph (see (e) of FIG. 2).

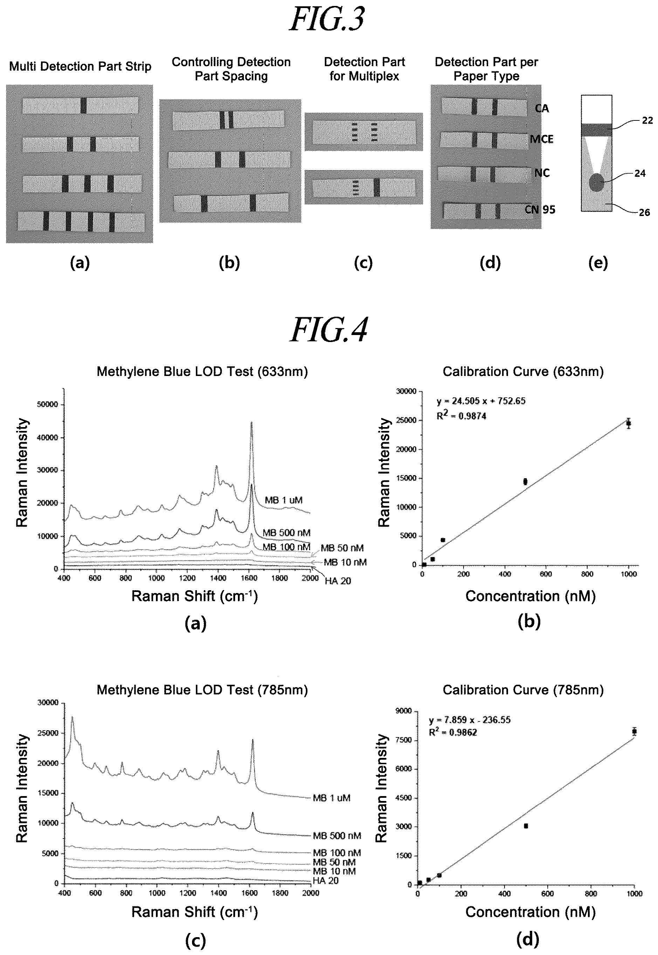

FIG. 3 schematically illustrates a lateral flow assay strip including polycrystalline structures in at least two detection parts of the test area according to one embodiment of the present disclosure. (a) of FIG. 3 illustrates a lateral flow assay strip including multi-detection part, (b) of FIG. 3 illustrates a lateral flow assay strip including a space-controlled multi-detection part, (c) of FIG. 3 illustrates a lateral flow assay strip including a polycrystalline structure of multiplex, (d) of FIG. 3 illustrates a lateral flow assay strip including a polycrystalline structure prepared with various types of strip members, and (e) of FIG. 3 illustrates a lateral flow assay strip including a detection part in a vertical direction and a detection circle in a circular form.
FIG. 4 are graphs showing the measurement results of SERS signals of Methylene Blue at various concentrations using a lateral flow assay strip having a polycrystalline structure in a detection line according to one embodiment of the present disclosure.
FIG. 5 are images showing the uniformity of SERS signals only in an area, where a polycrystalline structure is formed, through plasmonic mapping of a detection line of a lateral flow assay strip having a polycrystalline structure in a detection according to one embodiment of the present disclosure.
FIG. 6 are images showing fluorescence signals of Methylene Blue at various concentrations using a lateral flow assay strip having a polycrystalline structure in a detection line according to one embodiment of the present disclosure.
FIG. 7A are photographs of a lateral flow assay strips including a detection part of a heterogeneous material provided with a detection line including a polycrystalline structure and a detection line including a graphene oxide according to one embodiment and SEM images of each detection line.
FIG. 7B are figures illustrating SERS signal and fluorescence signal measured by using a lateral flow assay strip of heterogeneous materials provided with a detection line including a polycrystalline structure and a detection line including a graphene oxide layer.
FIG. 8 is a schematic diagram schematically illustrating use of a lateral flow assay strip including a polycrystalline structure according to one embodiment as a Matrix Metalloproteinase (MMP) sensor.
(a) in FIG. 9 are images of Raman mapping with a probe peptide for MMP of the first detection part, a vertical detection line including a polycrystalline structure, per fixed concentration according to one embodiment of the present disclosure.
(b) to (d) in FIG. 9 are graphs showing Raman spectra per concentration, a calibration curve, and signal uniformity in the area of first detection part of a peptide probe for MMP of the first detection part, the vertical detection line including a polycrystalline structure, according to one embodiment of the present disclosure.

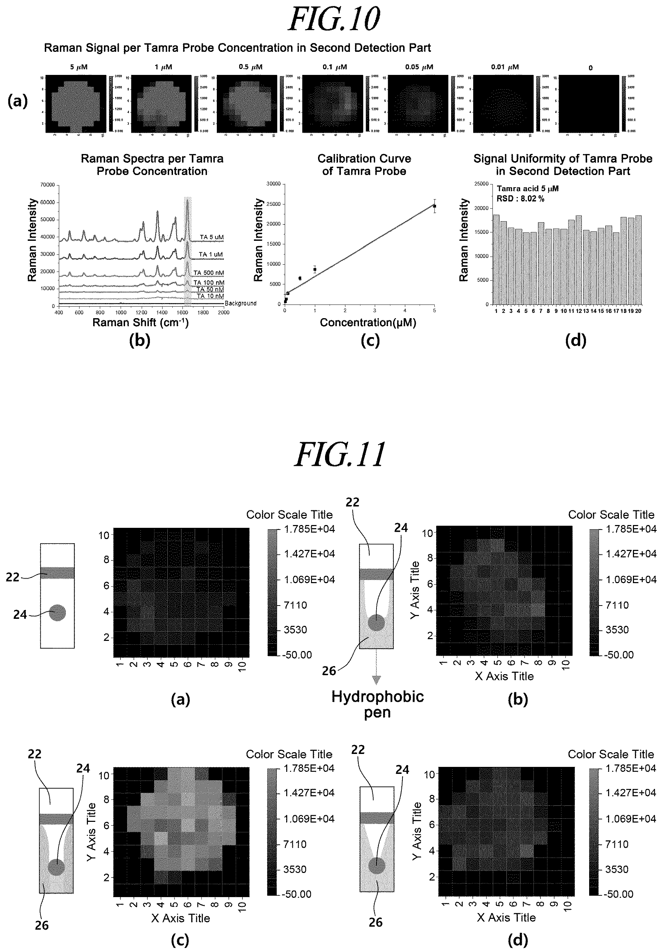

(a) in FIG. 10 are images of Raman mapping per concentration of a Tamra probe in the second detection part, the detection circle, positioned behind and detached from the first detection part including the polycrystalline structure according to one embodiment of the present disclosure.
(b) to (d) in FIG. 10 are graphs of Raman spectra per concentration, a calibration curve, and signal uniformity in the area of the second detection part of a Tamra probe of the second detection part, the detection circle, including the polycrystalline structure according to one embodiment of the present disclosure.
FIG. 11 are images of comparison of Raman signal size of detection per design of hydrophobically treated area around the second detection part, the detection circle, including the polycrystalline structure according to one embodiment of the present disclosure.

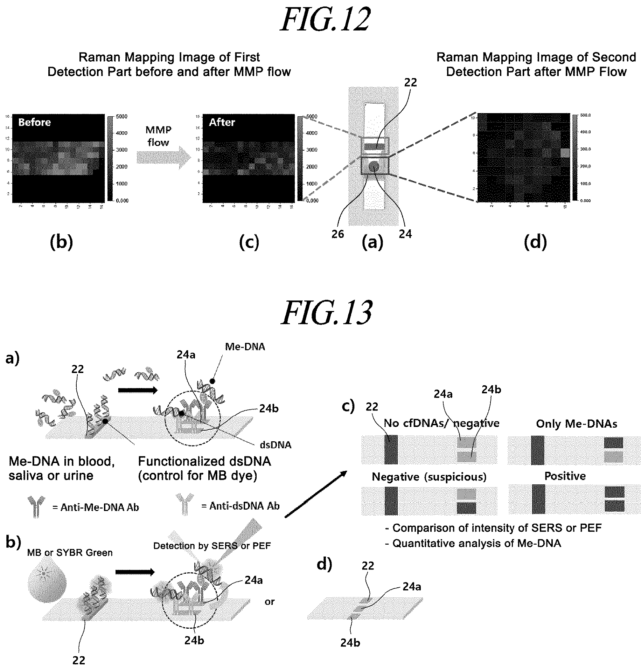

(a) in FIG. 12 is a photograph showing a lateral flow assay strip including a first detection part, a vertical detection line, and a second detection part, a detection circle, of a polycrystalline structure according to one embodiment of the present disclosure.
(b) and (c) of FIG. 12 are Raman mapping images of the first detection part before and after flow of a specimen including MMP through the lateral flow assay strip of (a) of FIG. 12.
(d) of FIG. 12 is a Raman mapping image of the second detection part after flow of a specimen including MMP through the lateral flow assay strip of (a) of FIG. 12.
FIG. 13 is a schematic diagram schematically illustrating use of a lateral flow assay strip including a polycrystalline structure as a Me-DNA sensor according to one embodiment of the present disclosure.
(a) of FIG. 14 are images showing fluorescence signal measurement results according to various intercalation dyes when the lateral flow assay strip including a polycrystalline structure according to one embodiment is used as a Me-DNA sensor.
(b) of FIG. 14 are images showing Raman mapping results according to various intercalation dyes when the lateral flow assay strip including a polycrystalline structure according to one embodiment of the present disclosure is used as a Me-DNA sensor.

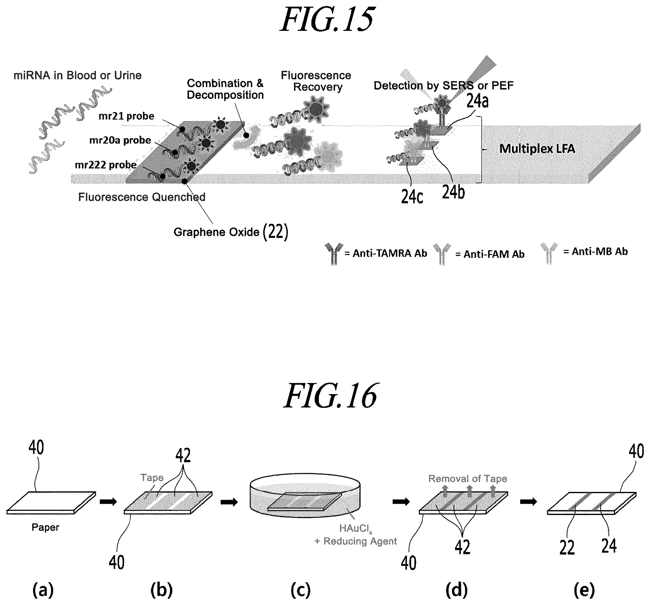

FIG. 15 is a schematic diagram schematically illustrating use of the lateral flow assay strip including a polycrystalline structure according to one embodiment of the present disclosure as a miRNA sensor.
FIG. 16 is a schematic diagram schematically illustrating a preparation method (method A) of a lateral flow assay strip including a polycrystalline structure by forming a polycrystalline structure after patterning according to one embodiment of the present disclosure.

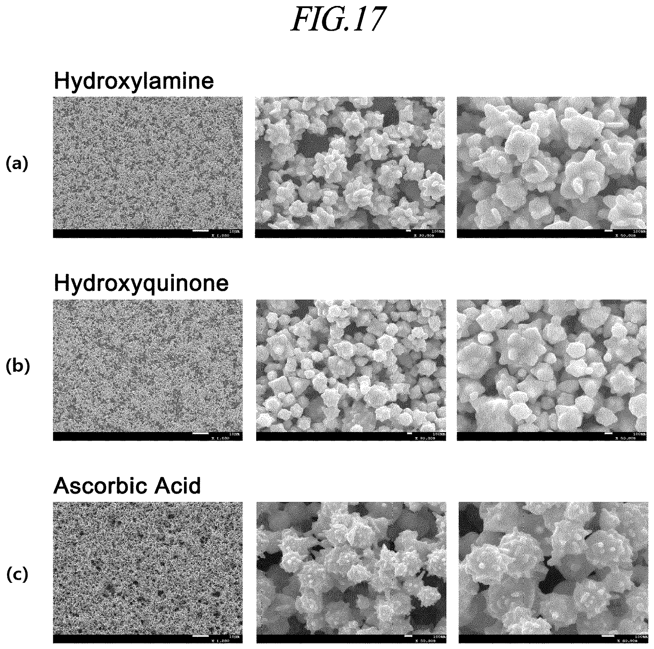

FIG. 17 is an image showing comparisons of formations of polycrystalline structures formed per type of a reducing agent when CA is used as the strip member for preparation according to the preparation method (method A) of the lateral flow assay strip including a polycrystalline structure according to one embodiment of the present disclosure.

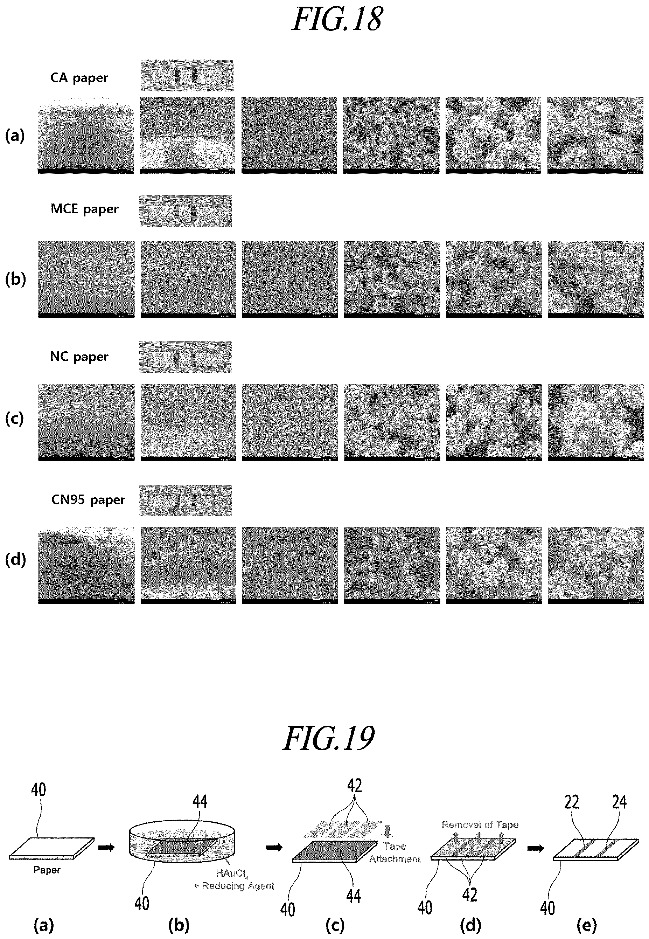

FIG. 18 are images showing comparisons of shapes of polycrystalline structures formed per type of a strip member while the same reducing agent is used in a preparation method (method A) of a lateral flow assay strip including a polycrystalline structure according to one embodiment of the present disclosure.
FIG. 19 is a schematic diagram schematically illustrating a preparation method (method B) of preparing a lateral flow assay strip including a polycrystalline structure by forming and patterning a polycrystalline structure according to one embodiment of the present disclosure.

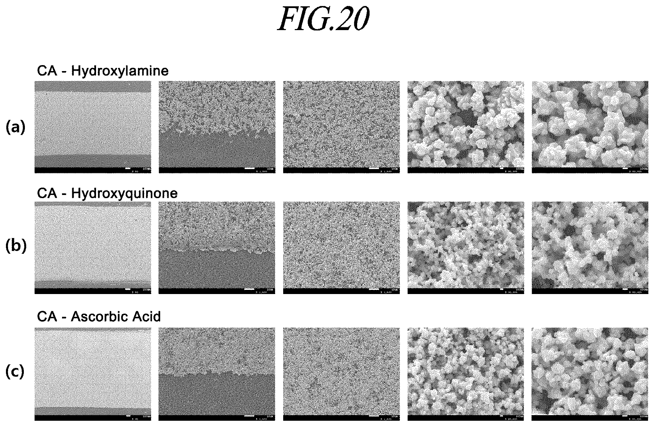

FIG. 20 are images showing comparison of shapes of polycrystalline structures per type of a reducing agent in the preparation method (method B) of preparing a lateral flow assay strip including a polycrystalline structure by forming and patterning a polycrystalline structure according to one embodiment of the present disclosure.

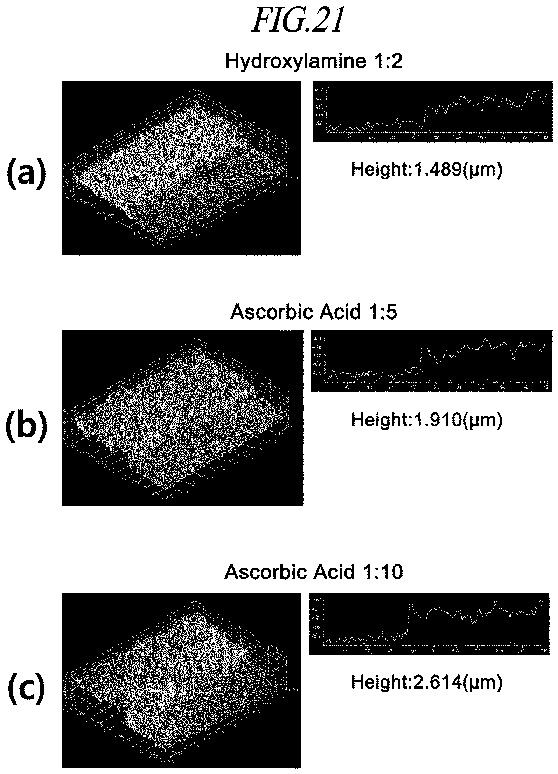

FIG. 21 are images showing comparisons of heights of detection parts of a strip member formed per reducing agent type and combination ratio of a reducing agent in a preparation method (method B) of a lateral flow assay strip including a polycrystalline structure by forming and patterning a polycrystalline structure according to one embodiment of the present disclosure.

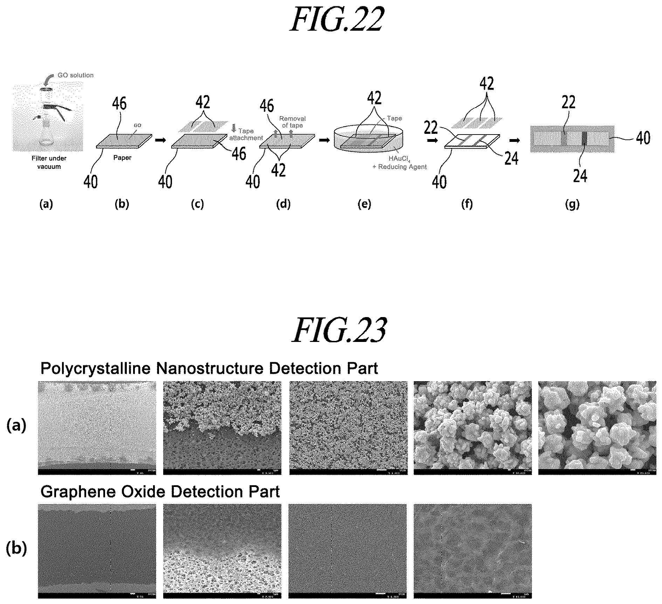

FIG. 22 is a schematic diagram schematically illustrating a preparation method (method C) of a lateral flow assay strip including a detection part of a heterogeneous material provided with a detection line including a polycrystalline structure (polycrystalline nanostructure detection part) and a detection line including graphene oxide (graphene oxide detection part) according to one embodiment of the present disclosure.
(a) of FIG. 23 are SEM images of a polycrystalline nanostructure detection part of lateral flow assay strip prepared according to FIG. 22.
(b) of FIG. 23 are SEM images of a graphene oxide detection part of lateral flow assay strip prepared according to FIG. 22.

### DETAILED DESCRIPTION

Purposes, specific advantages, and noble characteristics of the present disclosure will become much clearer through the detailed description and embodiments, hereinafter, in relation to the attached figures.

First, terms or words used in this specification and claims should not be construed as their general dictionary meanings, but as a meaning and a concept that correspond to the technical idea of the present disclosure based on the principle that an inventor may appropriately define a concept of a term in order to explain his/her invention to the best method.

In the specification, when elements, such as a layer, a part, or a substrate, are described to be "on," "connected to," or "coupled to" another element, such descriptions may refer that the elements are directly "on," "connected to," or "coupled to" the another element, or that at least another element may be interposed between the two elements. In the contrary, when an element is described to be "directly on," "directly connected to," or "directly coupled to" another element, there is no other element interposed between the two elements.

Terms used in the specification are merely used for explanation of specific embodiments and are not intended to limit the present disclosure. Singular expressions include plural expressions unless the context clearly indicates otherwise.

Terms, such as "include" or "have," described in the specification are merely for indicating presence of characteristics, numbers, steps, actions, elements, parts or combinations thereof. The terms should not be interpreted as preliminarily excluding possibilities of presence or addition of at least one other characteristic, number, action, element, part, or combination thereof.

In the specification, a description that a part "includes" an element refers that, unless there is a specific contrary description, the element may be further comprised, and other elements are not excluded. Also, in the entire specification, the description of being "on" an object refers to a state of being disposed above or below a part of the object and does not necessarily mean the state of being above based on the gravitational direction.

The present disclosure may be applied with various variations and have several embodiments. Accordingly, specific examples are illustrated in figures and explained in detail in the detailed descriptions. Nonetheless, these are not to limit the present disclosure to specific embodiments. Instead, these should be understood that the disclosure includes every variation, and equivalent to substituent included in the scope of ideas and technology of the present disclosure. In explanation of the present disclosure, when detailed descriptions about a related, known technology may obscure the gist of the present disclosure, the detailed descriptions thereof are omitted.

Hereinafter, embodiments of the present disclosure are explained in detail with reference to the attached figures. In explanation with reference to the attached figures, the same or corresponding components shall be assigned with the same reference figure numeral, and redundant descriptions thereof shall be omitted.

FIG. 1 schematically illustrates a lateral flow assay strip including a polycrystalline structure in a detection line according to one embodiment of the present disclosure.

Referring to FIG.1, a lateral flow assay strip 1 is consisted of a specimen area 10; a test area 20; and an absorption area 30 positioned in one direction according to one aspect of the present disclosure.

The specimen area 10 is an area for introduction of a specimen that may be configured to have a groove for containing a certain amount of a specimen.

The test area 20 may be positioned downstream of the specimen area 10 and may include at least one detection part 22, 24, where a biochemical reaction occurs between a target included in the specimen and a probe as the specimen flows. There is no specific limitation for the biochemical reaction as long as a target may be detected. The biochemical reaction includes all types of reactions such as an immune reaction or a chemical reaction (enzyme reaction and DNA reaction).

In the present disclosure, the detection parts 22, 24 are characterized in being consisted of a plurality of clusters of nanoparticles to include a polycrystalline structure having a plurality of grain boundaries.

The absorption area 30 is positioned downstream of the test area 20 so that it is configured to maintain a lateral flow by absorbing an excess amount of specimen.

(a) of FIG. 2 illustrates a detection part prepared with a strip member of cellulose acetate according to one embodiment of the present disclosure. (b) to (e) of FIG. 2 illustrate a SEM photograph of a detection part in (a) of FIG. 2 (see (b) of FIG. 2), a polycrystalline verification result (see (c) of FIG. 2), a nanoparticle formation mechanism (see (d) of FIG. 2), and a nanoparticle distribution graph (see (e) of FIG. 2). FIG. 3 schematically illustrates a lateral flow assay strip including a polycrystalline structure in at least two detection parts of the test area according to one embodiment of the present disclosure. (a) of FIG. 3 illustrates a lateral flow assay strip including multi-detection part, (b) of FIG. 3 illustrates a lateral flow assay strip including a space-controlled multi-detection part, (c) of FIG. 3 illustrates a lateral flow assay strip including a detection part for multiplex with two or more detection parts of vertical and/or horizontal direction aligned parallel to each other or in a row, (d) of FIG. 3 illustrates a lateral flow assay strip including a polycrystalline structure prepared with various types of strip members, and (e) of FIG. 3 illustrates a lateral flow assay strip including a detection part in a vertical direction and a detection circle in a circular form.

Referring to (a) to (e) of FIG. 2 and FIG. 3, it can be demonstrated that a polycrystalline is formed with a plurality of clusters of nanoparticles on a strip member of cellulose acetate (see (a) to (e) of FIG. 2) and other various strip members (see (d) of FIG. 3).

Furthermore, referring to (d) of FIG. 2, although not limited thereto, it is shown that an Au precursor among noble metals form nanoparticles to form a cluster and specifically that these directly grow on a strip member by using a solution process. Specifically, the clustered nanoparticles form small size grains of a polygonal structure, and then the particles gradually grow by oriented attachment. Accordingly, a polycrystalline structure having a plurality of grain boundaries (GB) is formed. Although not limited thereto, a particle in a noble metal coral form may grow to a micro size on average (see (e) of FIG. 2). When used as a lateral flow assay strip, a polycrystalline structure having a plurality of grain boundaries may dramatically improve signal intensity and signal uniformity as scattering increases at the plurality of grain boundaries.

Although not limited thereto, the nanoparticles may be interconnected in a branch structure, and the polycrystalline structure may have an average diameter of 1µm to 100µm. A polycrystalline structure of the detection part of the lateral flow assay strip of the present disclosure may have a polycrystalline structure grown by a solution process and interconnected in a branch form with an average diameter of 0.1µm to 100µm. Although not limited thereto, an average diameter of the polycrystalline structure may be suitable for improving signal intensity and signal uniformity within the said range.

There is no specific limitation to the strip member applicable to the present disclosure, but various materials may be used. Although not limited thereto, referring to (d) of FIG. 3, the strip member may be consisted of at least one species of Cellulose Acetate (CA), Mixed Cellulose Ester (MCE), Nitro Cellulose (NC), CN95, and a filter membrane composed of a synthetic polymer. Although not limited thereto, the filter membrane may be composed of polyesthersulfone, polycarbonate, glass fiber, nylon, or polytetrafluoroethylene.

Referring to FIG. 3, although not limited thereto, the first detection part and the second detection part 22, 24 may include at least one of the following.
i) at least two vertical detection lines positioned perpendicular to the direction of the specimen flow (see (a) of FIG. 3);
ii) at least two horizontal detection lines positioned parallel to the direction of the specimen flow (see (c) of FIG. 3); and
iii) at least one each of a vertical detection line positioned perpendicular to the direction of the specimen flow and a horizontal detection line positioned parallel to the direction of the specimen flow (see (c) of FIG. 3).

As above, there may be various combinations of at least one vertical detection line and at least one horizontal detection line.

In addition, the detection part may include at least one each of a first detection part 22, which is a vertical detection line positioned perpendicular to the direction of the specimen flow, and a second detection part 24, which is a detection part in a circle form positioned behind the first detection part 22, the vertical detection line, with respect to the direction of the specimen flow (see (e) of FIG. 3). That is, there may be various combinations such as one first detection part 22, the vertical detection line, with two or more second detection parts, the detection circle, or two or more first detection parts, the vertical detection line, with one second detection part, the detection circle.

There is no specific limitation to the detection circle of second detection part 24 as long as it is in a form to further concentrate a trace amount of a specimen. Accordingly, although not limited thereto, the detection circle may be modified into various forms, such as an elliptical shape or a polygonal shape, other than a circular form.

Although not limited thereto, a hydrophobically treated area 26 may be provided partially around the second detection part 24, the detection circle, so that a specimen that passed the first detection part 22, the vertical detection line, is gathered to the second detection part 24, the detection circle. Although not limited thereto, it may be suitable for signal enhancement that a part of the hydrophobically treated area 26, where a specimen flows between the first detection part 22, the vertical detection line, and the second detection part 24, the detection circle, is minimized while a part where the specimen flows and a part where the second detection part 24, the detection circle, is in contact are maintained around between 1/3 to 1/4 of the diameter of the second detection part 24, the detection circle(see (e) of FIG. 3). In addition, the hydrophobically treatment may be easily treated by using a hydrophobic pen.

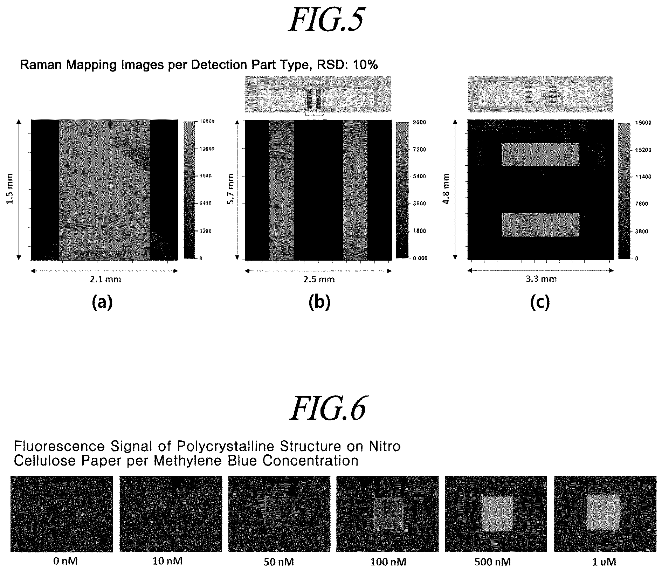

FIG. 4 are graphs showing the measurement results of SERS signals of Methylene Blue at various concentrations using a lateral flow assay strip having a polycrystalline structure in a detection line according to one embodiment of the present disclosure. FIG. 5 are images showing the uniformity of SERS signals only in an area, where a polycrystalline structure is formed, through plasmonic mapping of a detection line of a lateral flow assay strip having a polycrystalline structure in a detection according to one embodiment of the present disclosure. FIG. 6 are images showing fluorescence signal of Methylene Blue at various concentrations using a lateral flow assay strip having a polycrystalline structure in a detection line according to one embodiment of the present disclosure.

Referring to FIGS. 4 to 6, although not limited thereto, a lateral flow assay strip of the present disclosure may be for Surface Enhanced Raman Spectroscopy (SERS), fluorescence, or Plasmon-Enhanced Fluorescence (PEF) assay. A lateral flow assay strip of the present disclosure may include a plasmonic polycrystalline structure to allow a precise on-site diagnosis with high sensitivity when a non-label biological specimen is analyzed through SERS, fluorescence, or PEF. Specifically, as shown in FIGS. 4 and 5, a quantitative analysis may be provided with high reliability by using SERS signal at both wavelengths of 633 nm and 785nm, and, as shown in FIG. 6, a qualitative analysis may be provided by using PEF. Although not limited thereto, it may be more suitable for SERS or fluorescence analysis depending on a type of a strip member. For example, CA is more suitable for SERS analysis, and NC is more suitable for fluorescence analysis.

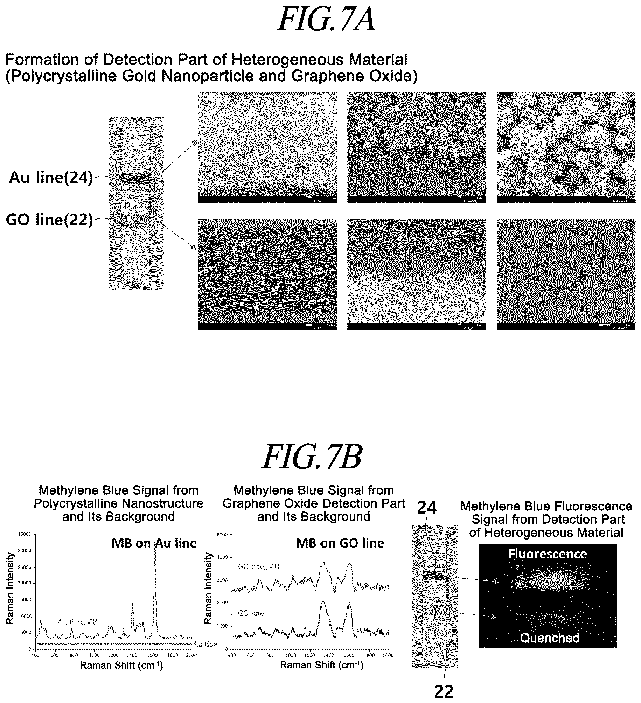

FIG. 7A are photographs of a lateral flow assay strip including a detection part of a heterogeneous material provided with a detection line including a polycrystalline and a detection line including a graphene oxide layer according to one embodiment and SEM images of each detection line. FIG. 7B are figures illustrating SERS signal and fluorescence signal measured by using a lateral flow assay strip of heterogeneous materials provided with a first detection part 22, a detection line including a polycrystalline structure, and a second detection part 24, a detection line including a graphene oxide layer.

As shown in FIGS. 7A and 7B, the first detection part and the second detection part 22, 24 may include a detection line including heterogeneous materials. There is no specific limitation to the heterogeneous material as long as it is a material for a control for a precise detection of a target by being composed of a material other than a polycrystalline structure, or a material for simultaneous detection of various species of targets.

FIGS. 7A and 7B exemplarily illustrate a lateral flow assay strip including a first detection part 22, a detection line (GO-line) including a graphene oxide layer, and a second detection part 24, a detection line (Au line) including a polycrystalline structure, so that it may be used as a multiplex sensor.

As shown in the left graph in FIG. 7B, a specimen may be analyzed with SERS signal at the second detection part 24, a detection line (Au line) including a polycrystalline structure.

In addition, although not limited thereto, the first detection part 22, a detection line (GO-line) including a graphene oxide layer, may be combined with a fluorescent molecule-labeled genetic probe, and the second detection part 24, a detection line (Au line) including a polycrystalline structure, may be combined with an antibody with which the fluorescent molecule may be combined.

Therefore, as shown in the right image in FIG. 7B, as a specimen flows, a fluorescent molecule with fluorescence quenched as it combines with a genetic probe in the first detection part 22, the detection line (GO line) including a graphene oxide layer, may recover fluorescence when a genetic probe combines with a miRNA target molecule and is detached from a genetic probe. The fluorescence signal may be detected as the fluorescence molecule combines with an antibody in the second detection part 24, the detection line (Au line) including a polycrystalline molecule. Angew. Chem Int. Ed, 48(26) (2009). Pp 4785 may be referred for a detailed mechanism of the fluorescence recovery.

Although not limited thereto, an antibody attached to each of the second detection part 24 may be anti-FAM, anti-TAMRA, anti-Methylene Blue, anti-Cy5, anti-Cy3, anti-Alexa Fluor 488, anti-Rhodamine Red, anti-Texas red, or anti-Fluorescein/Oregon Green.

Although not limited thereto, the specimen may be selected from at least one species of urine, saliva, sweat, blood and tear drop. At least one species selected from a cell, a metabolite, a protein, a nucleic acid, DNA, RNA, an enzyme, an organic molecule, a virus, an extracellular vesicle, a microvesicle, an exosome, and fat in the said specimen may be detected.

FIG. 8 is a schematic diagram schematically illustrating use of a lateral flow assay strip including a polycrystalline structure according to one embodiment as a Matrix Metalloproteinase (MMP) sensor.

Referring to FIG. 8, the detection part is provided with a first detection part 22, a vertical detection line positioned perpendicular to a direction of a flow of a specimen, such as a tear drop or blood, and a second detection part 24, a detection circle. The first detection part 22, which is first to contact the specimen, may have a polycrystalline structure combined with a probe peptide, which may be decomposed by MMP, and the second detection part 24, a detection circle positioned behind the first detection part 22 with respect to the direction of the specimen flow, may be combined with a probe for detecting the decomposed probe peptide so that the lateral flow assay strip of the present disclosure may be used as a MMP sensor.

MMP may induce an immune reaction as a biomarker of cancer. However, it is present in blood, saliva, or urine to the level of ng/mL so that it is not easy to be detected. According to the said configuration, as the specimen flows, the probe peptide combined with the polycrystalline structure of the first detection part 22 is decomposed by MMP, and the decomposed probe peptide may combine with the polycrystalline structure of the second detection part 24 so that it may be detected by SERS or PEF analysis. In addition, the probe peptide combined with the polycrystalline structure may combine with an antibody, the probe for detecting the probe peptide, so that it may be detected by SERS or PEF analysis.

Although not limited thereto, the combination of the polycrystalline structure and the probe peptide in the first detection part 22 may be by a dye-labeled peptide-SH having a sequence of Mca-Lys-Pro-Leu-Gly-Leu-Dap (Dnp)-Ala-Arg.

Although not limited thereto, the antibody to combine with the probe peptide in the second detection part 24 may be Anti-Tamra antibody.

A method to attach an antibody and the technique to decompose a dye-labeled probe peptide by MMP may use various known techniques. For reference, Fransiska S. H. Krismastuti, Stephanie Pace and Nicolas H. Voelcker, Adv. Funct. Mater. 2014, 24, 3639-3650 and Ying Ye, Yuancai Ge, Qingwen Zhang, Meiling Yuan, Yu Cai, Kang Li, Yang Li, Ruifeng Xie, Changshun Xu, Danfeng Jiang, Jia Qu, Xiaohu Liu, and Yi Wang Ying Ye, Adv. Sci. 2022, 2104738 may be referred to.

(a) in FIG. 9 is an image of Raman mapping with a probe peptide for MMP of the first detection part 22, a vertical detection line including a polycrystalline structure, per fixed concentration according to one embodiment of the present disclosure. (b) to (d) in FIG. 9 are graphs showing Raman spectra per concentration, a calibration curve, and signal uniformity of a peptide probe for MMP in the area of first detection part 22, the vertical detection line including a polycrystalline structure, according to one embodiment of the present disclosure.

Referring to (a) to (d) in FIG. 9, it can be demonstrated that Raman signals may be enhanced in a concentration-dependent manner of the probe peptide decomposed by MMP for SERS analysis. In addition, although not limited thereto, probe peptide decomposed by MMP may be detected even in a trace amount of concentration of 0.5 µM while the signal uniformity is also high to secure reliability of the test results.

(a) in FIG. 10 are images of Raman mapping by concentration of a Tamra probe in the second detection part 24, the detection circle, positioned behind and detached from the first detection part 22 including the polycrystalline structure according to one embodiment of the present disclosure. (b) to (d) in FIG. 10 are graphs of Raman spectra per concentration, a calibration curve, and signal uniformity in the area of the second detection part 24 of a Tamra probe of the second detection part 24, the detection circle, including the polycrystalline structure according to one embodiment of the present disclosure.

Referring to (a) to (d) in FIG. 10, it can be demonstrated that Raman signals may be enhanced in a concentration-dependent manner of the Tamra probe peptide for SERS analysis. In addition, although not limited thereto, Tamra probe may be detected even in a trace amount of concentration of 10 nM while the signal uniformity is also high to secure reliability of the test results.

As above, referring to FIGS. 9 and 10, it may be used as a MMP sensor with excellent sensitivity and reliability.

FIG. 11 are images of comparison of Raman signal size of detection per design of hydrophobically treated area 26 around the second detection part 24, the detection circle, including the polycrystalline structure according to one embodiment of the present disclosure.

Referring to FIG. 11, although not limited thereto, it may be effective for signal enhancement that a hydrophobically treated area 26 is provided partially around the second detection part 24, the detection circle, so that a specimen that passed the first detection part 22, the vertical detection line, is gathered to the second detection part 24, the detection circle. Although not limited thereto, the hydrophobically treated area 26 may be preferably formed to minimize an area of a specimen flow between the first detection part 22, the vertical detection line, and the second detection part 24, the detection circle (see (a) to (c) of FIG. 11). In addition, it is suitable for signal enhancement that a part where the specimen flows and a part where the second detection part 24, the detection circle, is in contact are maintained around between 1/3 to 1/4 of the diameter of the second detection part 24, the detection circle(see (c) and (d) of FIG. 11).

(a) in FIG. 12 is a photograph showing a lateral flow assay strip including a first detection part 22, a vertical detection line, and a second detection part 24, a detection circle, including a polycrystalline structure according to one embodiment of the present disclosure. (b) and (c) of FIG. 12 are Raman mapping images of the first detection part 22 before and after flow of a specimen including MMP through the lateral flow assay strip of (a) of FIG. 12. (d) ofFIG. 12 is a Raman mapping image of the second detection part 24 after flow of a specimen including MMP through the lateral flow assay strip of (a) of FIG. 12.

Referring to (b) and (c) of FIG. 12, it can be confirmed that the SERS signals are decreased after the flow of the specimen including MMP through the first detection part 22 than before the flow. Accordingly, MMP may be detected based on decrease of SERS signal according to concentration change of probe peptide through decomposition by MMP. In addition, referring to (d) of FIG. 12, MMP may be detected based on increase of SERS signal of the second detection part 24.

FIG. 13 is a schematic diagram schematically illustrating use of a lateral flow assay strip including a polycrystalline structure as a Me-DNA (methylated DNA) sensor according to one embodiment of the present disclosure.

Referring to FIG. 13, the detection part is provided with a first detection part 22, a vertical detection line, positioned perpendicular to a direction of flow of a specimen, such as blood, saliva or urine, a second detection part 24a, a horizontal detection line, positioned behind the first detection part and parallel to a direction of flow of a specimen, and a third detection part 24b, a second horizontal detection line. The second detection part 24a and the third detection part 24b may be positioned parallel to each other. A polycrystalline structure of the first detection part 22, the vertical detection line, may be combined with a gene to react with an intercalation dye as a first control, a polycrystalline structure of the second detection part 24a, a first horizontal detection line, may be combined with an antibody to combine with Me-DNA, and a polycrystalline structure of the third detection part 24b, the second horizontal detection line, may be combined with the genetic probe so that the lateral flow assay strip of the present disclosure can be used as a Me-DNA sensor.

Because Me-DNA, a biomarker of cancer, is present in blood, saliva, or urine in fM - nM, its detection is not easy.

Although not limited thereto, the gene may be dsDNA, dsRNA, aptamer, ssDNA or ssRNA.

The intercalation dye includes all types of dyes capable of intercalation into the gene. For example, the intercalation dye includes Methylene Blue, SYBR Green, EVA Green, ROX dye, Thiazole Red, TOTO, and VeriFluor far-red dye.

In the present disclosure, there is no specific limitation to the genetic probe or the probe as long as the presence of a material, such as a gene, can be verified. Although not limited thereto, the genetic probe may be an antibody or a dye to combine or react with the gene.

Referring to (b) in FIG. 13, the first detection part 22, the vertical detection line, is the first control combined with a gene to react with the intercalation dye, and the second detection part 24a, the first horizontal detection line, is combined with an antibody to combine with Me-DNA so that Me-DNA can be combined. The third detection part 24b, the second horizontal detection line, is the second control, which is combined with an antibody to combine with dsDNA so that dsDNA can be combined.

Referring to (b) and (c) in FIG. 13, after specimen flows through the first detection part 22, the first vertical detection line and the first control, MB or SYBR Green dye is flown to occur a reaction. That is, in (c) in FIG. 13, the first detection part 22 is marked in black as an indication that a reaction has occurred.

Then, if Me-DNA is present in a specimen, it combines with a Me-DNA antibody present in the second detection part 24a, the first horizontal detection line, so that Me-DNA can be detected by SERS or PEF. In this case, the second detection part 24a is marked in black, and the case that Me-DNA is not detected is marked in grey. In addition, a reaction also occurs in the third detection part 24b, the second horizontal detection line and the second control, combined with a dsDNA antibody, and the third detection part 24b is also marked in black. This case is shown as the bottom right schematic diagram of FIG. 13 (c), and it may be determined to be Me-DNA positive.

Meanwhile, if a reaction does not occur in both of the second detection part 24a, the first horizontal detection line, and the third detection part 24b, the second horizontal detection line, it is shown as top left schematic diagram in (c) of FIG. 13, and it may be determined to be negative. In addition, even in the case (bottom left schematic diagram in (c) of FIG. 13) that a reaction occurs only in the third detection part 24b, the second horizontal detection line and the second control, and a reaction does not occur in the second detection part 24a, the first horizontal detection line, it can be determined that it is Me-DNA negative. Meanwhile, in the case (top right schematic diagram in (c) of FIG. 13) that a reaction occurs in the second detection part 24a, the first horizontal detection line, but a reaction does not occur in the third detection part 24b, the second horizontal detection line and the second control, it can be determined that it is false positive.

Referring to (d) of FIG. 13, a design of a detection line may be variously changed. As shown in (d) of FIG. 13, the detection part may have the first detection part 22, the first horizontal detection line positioned parallel to a direction of flow of the specimen, the second detection part 24a, the second horizontal detection line, and the third detection part 24b, the third horizontal detection line, the first detection part, the second detection part, and the third detection part positioned parallel to one another.

The first detection part 22, the first horizontal detection line, may have a polycrystalline structure combined with a gene to react with a intercalation dye as a first control, the second detection part 24a, the horizontal detection line, may have a polycrystalline structure combined with an antibody to combine with Me-DNA, and the third detection part 24b, the third horizontal detection line, may have a polycrystalline structure combined with the genetic probe so that a lateral flow assay strip of the present disclosure may be used as a Me-DNA sensor.

(a) of FIG. 14 are an image showing fluorescence signal measurement results according to various intercalation dyes when the lateral flow assay strip including a polycrystalline structure according to one embodiment is used as a Me-DNA sensor. (b) of FIG. 14 is an image showing Raman mapping result according to various intercalation dyes when the lateral flow assay strip including a polycrystalline structure according to one embodiment of the present disclosure is used as a Me-DNA sensor.

As shown in (a) of FIG. 14, the level of generation of fluorescence signal is different depending on a type of the dye. However, fluorescence signal generally increased more in presence of DNA than in absence of DNA. Specifically, the images surrounded in a black box corresponds to the dyes that clearly show the effects of increasing fluorescence signals.

(b) of FIG. 14 are images showing the level of generation of Raman signals depending on presence of DNA per type of the dye. The images surrounded in a black box corresponds to the dyes that show decrease of Raman signals in presence of DNA.

Accordingly, as shown in (a) and (b) of FIG. 14, the levels of increase of fluorescence or decrease of Raman signal intensities are different depending on a type of the dye. However, quantitative evaluation may be available by using the tendency of increasing fluorescence or decreasing Raman signals.

FIG. 15 is a schematic diagram schematically illustrating use of the lateral flow assay strip including a polycrystalline structure according to one embodiment of the present disclosure as a miRNA sensor.

Referring to FIG. 15, the detection part has the first detection part 22, the first detection line formed with a graphene oxide layer, and at least two second detection parts 24, the second detection lines with formation of the polycrystalline structures, the first detection part 22, the first detection line, combined with a fluorescent molecule labeled genetic probe, the second detection parts, the second detection lines, combined with an antibody to react with the fluorescent molecule, so that the lateral flow assay strip of the present disclosure may be used as a multiplex sensor.

Detection of miRNA is not easy because miRNA is a biomarker of cancer present in blood or urine in the range of pM - nM or in the amounts of ng/mL. In the present disclosure, the fluorescence molecule labeled genetic probe refers to a genetic combined with a fluorescence molecule. Types of genetic probes may be a RNA probe, a DNA probe, or an aptamer.

Referring to FIG. 15, as a specimen, such as blood or urine, flows, a fluorescent molecule with fluorescence quenched as it combines with a genetic probe in the first detection part 22, the first detection line, may recover fluorescence when a genetic probe combines with a miRNA target molecule and is detached from a genetic probe. The fluorescence signal may be detected as the fluorescence molecule combines with an antibody in the second detection part 24, the second detection line.

As above, because various species of genes may be simultaneously detected in the at least two second detection parts 24, the second detection lines, in the present disclosure, the lateral flow assay strip may be used as a multiplex sensor. According to conventional methods, it has been impossible to fix antibodies to one line by position.

Although not limited thereto, an antibody to be attached to each of the second detection parts 24, the second detection line, may be anti-FMA, anti-TMARA, anti-Methylene Blue, anti-Cy5, anti-Cy5, anti-Alexa Fluor 488, anti-Rhodamine Red, anti-Texas Red, or anti-Fluorescein/Oregon Green.

FIG. 16 is a schematic diagram schematically illustrating a preparation method (method A) of a lateral flow assay strip including a polycrystalline structure by forming a polycrystalline structure after patterning according to one embodiment of the present disclosure.

As shown in FIG. 16, according to another aspect of the present disclosure, a preparation method (method A) of a lateral flow assay strip includes A-1) preparing a strip member for preparing a strip member 40 for lateral flow assay; A-2) attaching a tape 42 for attaching a tape to the strip member 40 except for an area to be patterned; A-3) forming a polycrystalline structure for forming a polycrystalline structure having a plurality of grain boundaries by soaking the strip member 40 in a composition including a solution of a noble metal precursor and a reducing agent to form a plurality of clusters of nanoparticles on the strip member 40; and A-4) removing the tape for removing the tape 42 from the strip member 40.

In the step of preparing the strip member (A-1) as illustrated in (a) of FIG. 16, the strip member 40 may be prepared with at least one species of Cellulose Acetate (CA), Mixed Cellulose Ester (MCE), Nitro Cellulose (NC), CN95, and filter membrane composed of a synthetic polymer. Although not limited thereto, the filter membrane may be prepared with polyesthersulfone, polycarbonate, glass fiber, nylon, or polytetrafluoroethylene.

In the step of attaching the tape (A-2) illustrated in (b) of FIG. 16, the tape 42 is attached to the strip member 40 except for the area to be patterned. That is, the tape 42 is attached to the area where a detection part including a polycrystalline structure is formed. Here, although not limited thereto, all types of a polymer-based tape with adhesive components above, such as a polyimide tape, a (regular) scotch tape, or a post-it tape may be used as the tape.

In the step of forming a polycrystalline structure (A-3) for forming a polycrystalline structure as illustrated in (c) of FIG. 16, a solution process proceeds by soaking the strip member 40 attached with the tape in a composition including the solution of the noble metal precursor and the reducing agent. Here, a plurality of clusters of nanoparticles are formed on the strip member 40 except for the area, where the tape is attached, so that the polycrystalline structure having a plurality of grain boundaries is formed. The area where the polycrystalline structure is formed becomes the detection part of the lateral flow assay strip.

Although not limited thereto, the polycrystalline structure may be formed by a solution process by using a composition including a noble metal precursor and a reducing agent while a ratio of the reducing agent to the noble metal precursor is 1:0.5 to 1:15.

Although not limited thereto, if the ratio of the reducing agent to the noble metal precursor is less than 1:0.5, a Raman spectroscopy system at various wavelengths cannot be used, and if the ratio is greater than 1:15, it may be difficult to obtain a lateral flow strip with good signal intensity and signal uniformity due to filmization.

Accordingly, the ratio of the reducing agent to the noble metal precursor in the composition of the present disclosure may be suitably 1:0.5 to 1:15 for preparing a substrate for spectroscopic analysis with good signal intensity and signal uniformity, and may be 1:0.5 to 1:12, 1:0.5 to 1:10, 1:1 to 1:10, 1:1 to 1:9, 1:1 to 1:8, 1:1 to 1:7, 1:1 to 1:6, 1:1 to 1:5, 1:1 to 1:4, or 1:1 to 1:3. In addition, the range may be somewhat different depending on the type of the reducing agent.

Although not limited thereto, in the composition of the present disclosure, the size of the polycrystalline structure formed on the substrate for spectroscopic analysis decreases and the density of the polycrystalline structure increases as the ratio of the reducing agent to the noble metal precursor increases.

Although not limited thereto, the nanoparticle may be consisted of at least one species of Au, Ag, and Pt, and Au may be the most suitable for enhancement of signal intensity and signal uniformity.

Although not limited thereto, the noble metal precursor may be selected from a group of HAuCl₄, AuCl, AuCl₂, AuCl₃, Na₂Au₂Cl₈, and NaAuCl₂, and at least one species of HAuCl₄ and NaAuCl₄ may be more suitable.

Although not limited thereto, the reducing agent may be at least one species of hydroxylamine (HA), ascorbic acid (AA), FeSO₄, and hydroxyquinone (HQ).

The step of removing the tape (A-4) illustrated in (d) of FIG. 16 is the step of removing the tape 42 except for the area with the polycrystalline structure formation. As the result, the first detection part and the second detection part 22, 24 including a polycrystalline structure on the strip member 40 is formed as shown in (e) of FIG. 16.

FIG. 17 is an image showing comparisons of shapes of polycrystalline structures formed per type of reducing agents when CA is used as the strip member for preparation according to the preparation method (method A) of the lateral flow assay strip including a polycrystalline structure according to one embodiment of the present disclosure.

As shown in FIG. 17, while the same strip member of CA is used, different reducing agents of HA ((a) of FIG. 17), HQ ((b) of FIG. 17), or AA ((c) of FIG. 17) are used. In these cases, shapes of the formed polycrystalline structures are somewhat different depending on the used type of a reducing agent. However, it can be verified that a polycrystalline structure is well formed for all cases.

FIG. 18 is an image showing shapes of polycrystalline structures formed per type of a strip member while the same reducing agent of HA is used in a preparation method (method A) of a lateral flow assay strip including a polycrystalline structure according to one embodiment of the present disclosure.

As shown in FIG. 18, while the same reducing agent of HA is used, different strip members of CA paper ((a) of FIG. 18), MCE paper ((b) of FIG. 18), NC paper ((c) of FIG. 18), or CN95 paper ((d) of FIG. 18) are used. In these cases, shapes of the formed polycrystalline structures are somewhat different depending on the used type of a strip member. However, it can be verified that a polycrystalline structure is well formed for all cases.

FIG. 19 is a schematic diagram schematically illustrating a preparation method (method B) of preparing a lateral flow assay strip including a polycrystalline structure by forming and patterning a polycrystalline structure according to one embodiment of the present disclosure.

As shown in FIG. 19, according to another aspect of the present disclosure, a preparation method of a lateral flow assay strip includes B-1) preparing a strip member for preparing a strip member 40 for lateral flow assay; B-2) forming a polycrystalline structure for forming a polycrystalline structure having a plurality of grain boundaries by soaking the strip member 40 in a composition including a solution of a noble metal precursor and a reducing agent to form a plurality of clusters of nanoparticles on the strip member; B-3) attaching a tape 42 to the strip member 40 except for an area to be patterned; and B-4) removing the tape 42 from the strip member 40.

In comparison to the preparation method (method A) of the lateral flow assay strip as shown in FIG. 16, there is a difference in that the patterning proceeds by attaching the tape 42 to the strip member 40 after forming the polycrystalline structure 44. Accordingly, the same description that matches with the preparation method of the lateral flow assay strip of FIG. 16 is omitted.

As illustrated in (b) of FIG. 19, the polycrystalline structure 44 is formed in the entire area of the strip member 40 during the step of forming a polycrystalline structure for forming the polycrystalline structure in the step B-2).

As illustrated in (c) of FIG. 19, patterning proceeds by attaching the tape 42 except for the area where a detection part is to be formed in the step of attaching the tape in the step B-3).

As illustrated in (d) of FIG. 19, the polycrystalline structure in the area, where the tape 42 is attached, is removed with the tape by the adhesiveness of the tape 42 in the step of removing the tape in the step B-4). As the result, the first detection part and the second detection part 22, 24 including at least two detection lines including a polycrystalline structure on the strip member are formed as shown in (e) of FIG. 19.

FIG. 20 are images showing comparison of shapes of polycrystalline structures per type of a reducing agents while a strip member of CA is used for preparation by the preparation method (method B) of the lateral flow assay strip including a polycrystalline structure according to one embodiment of the present disclosure.

As shown in FIG. 20, while the same strip member of CA is used, different reducing agents of HA ((a) of FIG. 20), HQ ((b) of FIG. 20), and AA ((c) of FIG. 20) are used. Shapes of the formed polycrystalline structures are somewhat different depending on the used type of a reducing agent. However, it can be verified that a polycrystalline structure is well formed for all cases.

FIG. 21 are images showing comparisons of heights of detection parts of a strip member formed per reducing agent type and combination ratio of a reducing agent in a preparation method of a lateral flow assay strip including a polycrystalline structure by forming and patterning a polycrystalline structure according to one embodiment of the present disclosure.

As shown in FIG. 21, it can be verified that the height of the detection part becomes different depending on the reducing agent type and the reducing agent combination ratio.

FIG. 22 is a schematic diagram schematically illustrating a preparation method (method C) of a lateral flow assay strip including a detection part of a heterogeneous material provided with a detection line including a polycrystalline structure and a detection line including graphene oxide according to one embodiment of the present disclosure.

Referring to FIG. 22, according to another aspect of the present disclosure, a preparation method of a lateral flow assay strip includes C-1) preparing a strip member for preparing a strip member 40 for lateral flow assay; C-2) forming a graphene oxide layer 46 on the strip member 40 by using a solution of graphene oxide; C-3) attaching a first tape for attaching a tape 42 to the strip member 40 except for an area for graphene oxide to be patterned; C-4) removing the first tape for removing the tape 42 from the strip member 40; C-5) attaching a second tape for attaching a tape 42 to the strip member 40 except for an area for a polycrystalline structure to be patterned; C-6) forming a polycrystalline structure for forming a polycrystalline structure having a plurality of grain boundaries by soaking the strip member 40 in a composition including a solution of a noble metal precursor and a reducing agent to form a plurality of clusters of nanoparticles on the strip member 40; and C-7) removing the second tape for removing the tape 42 from the strip member 40.

As shown in (a) and (b) of FIG. 22, a graphene oxide layer 46 in the step C-2) may be formed by filtering a graphene oxide solution on the strip member 40 under vacuum. Although not limited thereto, it may be preferable for quenching fluorescence signals to form the graphene oxide layer 46 with a graphene oxide solution in a concentration range of 0.1 mg/mL - 10 mg/mL.

As shown in (c) of FIG. 22, the step of attaching the first tape in the step C-3) is a step of patterning by using the tape 42 to form the detection part including the graphene oxide layer 46.

As shown in (d) of FIG. 22, the step of removing the tape in the step C-4) is a step of removing the graphene oxide except for the area of the detection part including the graphene oxide layer 46 by using the tape 42.

The step of attaching the second tape in the step C-5) is a step of patterning to form the detection part including a polycrystalline structure by using the tape 42.

As shown in (e) of FIG. 22, the step of forming the polycrystalline structure for forming the polycrystalline structure in the step C-6) is a step of forming the detection part including the polycrystalline structure in the area where the tape 42 is not attached.

As shown in (f) and (g) of FIG. 22, the detection part 22, 24 including detecting lines of heterogeneous members are formed by removing the tape 42 in the step of removing the second tape in the step C-7).

According to another aspect, a preparation method (method D) of a lateral flow assay system of the present disclosure includes D-1) preparing a strip member for preparing a strip member for lateral flow assay; D-2) forming a polycrystalline structure for forming a polycrystalline structure having a plurality of grain boundaries by soaking the strip member in a composition including a solution of a noble metal precursor and a reducing agent to form a plurality of clusters of nanoparticles on the strip member; D-3) attaching a first tape for attaching a tape to the strip member except for an area for the polycrystalline to be patterned; D-4) removing the first tape for removing the tape from the strip member; D-5) attaching a second tape for attaching a tape to the strip member except for an area for graphene oxide to be patterned; D-6) forming a graphene oxide layer by using a graphene oxide solution on the strip member; and D-7) removing the second tape for removing the tape from the strip member.

The method D is different from the method C illustrated in FIG. 22 in that the detection part including the nanoparticle structure is formed before formation of the detection part including a graphene oxide layer.

(a) of FIG. 23 are SEM images of a polycrystalline nanostructure detection part of lateral flow assay strip prepared according to FIG. 22. (b) of FIG. 23 are SEM images of a graphene oxide detection part of lateral flow assay strip prepared according to FIG. 22.

Referring to FIG. 23, it can be verified that both of the polycrystalline nanostructure detection part and the graphene oxide detection part are well formed.

In preparation methods of a lateral flow assay strip as above, a multi detection line and a heterogenous material detection line may be easily prepared by patterning the polycrystalline structure by a solution process while spacing of the detection lines may be effectively controlled.

While certain embodiments of the present disclosure have been described above, anyone ordinarily skilled in the art to which the present disclosure pertains shall appreciate that there may be a variety of modifications and permutations of the present disclosure without departing from the technical ideas and scopes of the present disclosure that are defined in the appended claims. Moreover, it shall be appreciated that these modifications and permutations are also included in the scope of the present disclosure.

### Description of Reference Numerals

100: lateral flow assay strip
10: specimen area
20: test area
22, 24, 24a, 24b, 24c: detection part
26: hydrophobically treated area
30: absorption area
40: strip member
42: tape

## Claims

1. A lateral flow assay strip comprising a strip member comprising a test area comprising at least one detection part, where a biochemical reaction occurs between a target included in a specimen and a probe as the specimen flows by a capillary action,
wherein the detection part is consisted of a plurality of clusters of nanoparticles to comprise a polycrystalline structure having a plurality of grain boundaries.

2. The lateral flow assay strip of claim 1,
wherein the strip member comprises a specimen area for introducing a specimen; a test area; and
an absorption area for absorbing an excess amount of a specimen to maintain a lateral flow, that are placed in one direction.

3. The lateral flow assay strip of claim 1,
wherein the lateral flow assay strip is for Surface Enhanced Raman Spectroscopy (SERS), fluorescence, or plasmon-enhanced fluorescence (PEF) analysis.

4. The lateral flow assay strip of claim 1,
wherein the nanoparticle is consisted of at least one species of Au, Ag and Pt.

5. The lateral flow assay strip of claim 1,
wherein the polycrystalline structure is combined with a genetic probe.

6. The lateral flow assay strip of claim 1,
wherein the detection part comprises at least one of the following:
i) at least two vertical detection lines positioned perpendicular to a direction of a specimen flow;
ii) at least two horizontal detection lines positioned parallel to a direction of a specimen flow; and
iii) at least one vertical detection line positioned perpendicular to a direction of a specimen flow and at least one horizontal detection line positioned parallel to a direction of a specimen flow.

7. The lateral flow assay strip of claim 1,
wherein the detection part comprises at least one vertical detection line positioned perpendicular to a direction of a specimen flow and at least one detection part in a form of a circle, an ellipse, or a polygonal shape positioned behind the vertical detection line with respect to the direction of the specimen flow.

8. The lateral flow assay strip of claim 7,
wherein a hydrophobically treated area treated with hydrophobic treatment partially around the detection part so that a specimen passed through the vertical detection line is gathered to the detection part.

9. The lateral flow assay strip of claim 1,
wherein the detection part further comprises a detection part of a heterogeneous material.

10. The lateral flow assay strip of claim 9,
wherein the detection part comprises a first detection part with formation of a graphene oxide layer and at least two second detection parts with formation of the polycrystalline structure,
wherein the first detection part is combined with a fluorescent particle labeled genetic probe with fluorescence quenched, and
wherein the second detection parts are combined with a probe to combine with the fluorescent particle to recover fluorescence as the genetic probe combines with a miRNA target molecule so that the lateral flow assay strip can be used as a multiplex sensor.

11. The lateral flow assay strip of claim 10,
wherein the probe attached to each second detection part is anti-FAM, anti-TAMRA, anti-Methylene Blue, anti-Cy5, anti-Cy3, anti-Alexa Fluor 488, anti-Rhodamine Red, anti-Texas Red, or anti-fluorescein/Oregon Green.

12. The lateral flow assay strip of claim 1,
wherein the strip member is consisted of at least one species of Cellulose Acetate (CA), Mixed Cellulose Ester (MCE), Nitro Cellulose (NC), CN95, and a filter membrane composed of a synthetic polymer.
The lateral flow assay strip of claim 1,

13. The lateral flow assay strip of claim 1,
wherein the target is selected from at least one species of cells, metabolites, proteins, nucleic acids, DNAs, RNAs, enzymes, organic molecules, viruses, extracellular vesicles, microvesicles, exosomes, and fat in urine, saliva, sweat, blood and tear drops.

14. The lateral flow assay strip of claim 1,
wherein the detection part is provided with a first detection part and a second detection part orderly positioned with respect to a direction of the specimen flow, wherein a polycrystalline structure of the first detection part, which first to be contact with the specimen, is combined with a probe peptide to be decomposed by Matrix Metalloproteinase (MMP), and
wherein the second detection part is combined with a probe for detecting the decomposed probe peptide, so that the lateral flow assay strip can be used as a MMP sensor.

15. The lateral flow assay strip of claim 1,
wherein the detection part is provided with a first detection part, and a second detection part and a third detection part positioned behind the first detection part, the second detection part and the third detection part positioned parallel to each other,
wherein a polycrystalline structure of the first detection part is combined with a gene reacting with an intercalation dye as a first control,
wherein a polycrystalline structure of the second detection part is combined with an antibody that can combine with Me-DNA, and
wherein a polycrystalline structure of the third detection part is combined with the genetic probe, so that the lateral flow assay strip can be used as a Me-DNA.

16. The lateral flow assay strip of claim 1,
wherein the detection part is provided with a first detection part, a second detection part and a third detection part horizontally positioned with respect to a direction of the specimen flow, the first detection part, the second detection part and the third detection part positioned parallel to one another,
wherein a polycrystalline structure of the first detection part is combined with a gene reacting with an intercalation dye as a first control,
wherein a polycrystalline structure of the second detection part is combined with an antibody that can combine with Me-DNA, and
wherein a polycrystalline structure of the third detection part is combined with the genetic probe, so that the lateral flow assay strip can be used as a Me-DNA sensor.

17. A preparation method of a lateral flow assay strip comprising:
A-1) preparing a strip member for preparing a strip member for lateral flow assay;
A-2) attaching a tape for attaching a tape to the strip member except for an area to be patterned;
A-3) forming a polycrystalline structure for forming a polycrystalline structure having a plurality of grain boundaries by soaking the strip member in a composition including a solution of a noble metal and a reducing agent to form a plurality of clusters of nanoparticles on the strip member; and
A-4) removing the tape for removing the tape from the strip member.

18. A preparation method of a lateral flow assay strip comprising:
B-1) preparing a strip member for preparing a strip member for lateral flow assay;
B-2) forming a polycrystalline structure for forming a polycrystalline structure having a plurality of grain boundaries by soaking the strip member in a composition including a solution of a noble metal precursor and a reducing agent to form a plurality of clusters of nanoparticles on the strip member;
B-3) attaching a tape for attaching a tape to the strip member except for an area to be patterned; and
B-4) removing the tape for removing the tape from the strip member.

19. A preparation method of a lateral flow assay strip comprising:
C-1) preparing a strip member for preparing a strip member for lateral flow assay;
C-2) forming a graphene oxide layer on the strip member by using a graphene oxide solution;
C-3) attaching a first tape for attaching a tape to the strip member except for an area for the graphene oxide to be patterned;
C-4) removing the first tape for removing the tape from the strip member;
C-5) attaching a second tape for attaching a tape to the strip member except for an area for a polycrystalline structure to be patterned;
C-6) forming a polycrystalline structure for forming a polycrystalline structure having a plurality of grain boundaries by soaking the strip member in a composition including a solution of a noble metal precursor and a reducing agent to form a plurality of clusters of nanoparticles on the strip member; and
C-7) removing the second tape for removing the second tape from the strip member.

20. A preparation method of a lateral flow assay strip comprising:
D-1) preparing a strip member for preparing a strip member for lateral flow assay;
D-2) forming a polycrystalline structure for forming a polycrystalline structure having a plurality of grain boundaries by soaking the strip member in a composition including a solution of a noble metal precursor and a reducing agent to form a plurality of clusters of nanoparticles on the strip member;
D-3) attaching a first tape for attaching a tape to the strip member except for an area for a polycrystalline structure to be patterned;
D-4) removing the first tape for removing the tape from the strip member;
D-5) attaching a second tape for attaching a tape to the strip member except for an area for graphene oxide to be patterned;
D-6) forming a graphene oxide layer on the strip member by using a graphene oxide solution; and
D-7) removing the second tape for removing the tape from the strip member.
